# EUROPEAN PATENT APPLICATION

(11) **EP 2 918 225 A1**
(43) Date of publication of application: **16.09.2015**
(21) Application number: 13852693.4
(22) Date of filing: 08.11.2013
(51) Int. Cl.: A61B 5/0245, A61B 5/00

(54) **BIOLOGICAL INFORMATION MEASUREMENT DEVICE AND INPUT DEVICE USING SAME**

(30) Priority: 12.11.2012 JP 2012247983
(71) Applicant: Alps Electric Co., Ltd., Tokyo 145-8501 (JP)
(72) Inventor: YAMASHITA, Tatsumaro, Tokyo 145-8501 (JP); KAMATA, Tomoya, Iwate-gun Iwate 020-0173 (JP)
(74) Representative: Klunker . Schmitt-Nilson . Hirsch
(86) International application number: PCT/JP2013/080265
(87) International publication number: WO 2014/073645

(57) **Abstract**

[Object] To provide in particular a biological information measurement device capable of stably measuring biological information with a high degree of accuracy, compared with the related art, and an input device utilizing that.

[Solution] A biological information measurement device 10 includes near-infrared cameras 2a and 2b capable of detecting pupils of an object person, a detection unit 11 capable of detecting pupil areas from pieces of image data obtained by the near-infrared cameras 2a and 2b, a luminance acquisition unit 12 that acquires luminances of skin areas serving as at least portions of the peripheries of the pupil areas, and a biological measurement unit 13 that measures biological information of the object person from the luminances.

## Description

### Technical Field

The present invention relates to a biological information measurement device capable of measuring biological information.

### Background Art

In PTL 1 described below, there is disclosed an invention relating to a biological activity measurement device capable of detecting the average luminance of a specific area such as an area between eyebrows or a forehead and obtaining biological information such as the pulse rate of a test subject, based on the average luminance.

### Citation List

### Patent Literature

PTL 1: Japanese Unexamined Patent Application Publication No. 2011-130996

### Summary of Invention

### Technical Problem

In the invention described in PTL 1, in order to identify the area between eyebrows, the two-dimensional coordinates or three-dimensional coordinates of a whole on a face image are acquired and the area between eyebrows is identified based on three feature points on the coordinates.

However, in the method of PTL 1, it is thought that since calculation of the area between eyebrows is performed from the whole face image, a load on a control unit becomes large. In addition, while the area between eyebrows is detected from the three feature points, it is thought that, depending on an object person, there is a person whose feature point is unlikely to appear. In addition, in such a case, it becomes difficult to detect the area between eyebrows, thereby eventually leading to a measurement variation or measurement error of biological information.

Therefore, the present invention solves the above-mentioned problem of the related art and an object thereof is to provide in particular a biological information measurement device capable of stably measuring biological information with a high degree of accuracy, compared with the related art, and an input device utilizing that.

### Solution to Problem

An input device in the present invention includes an image capturing unit capable of detecting a pupil of an object person,
a detection unit capable of detecting a pupil area from image data obtained by the image capturing unit, a luminance acquisition unit that acquires a luminance of a skin area serving as at least a portion of the periphery of the pupil area, and a biological measurement unit that measures biological information of the object person from the luminance of the skin area.

Since, in the present invention, the skin area made available for sensing a luminance is identified based on the pupil area, it is possible to stably obtain the skin area and accordingly it is possible to stably obtain the luminance of the skin area with a high degree of accuracy. Therefore, it is possible to stably obtain, with a high degree of accuracy, the biological information obtained based on the luminance of the skin area. It is possible to correctly detect the pupil without fail unlike the other part of the face of the object person. Therefore, in contrast, in a case where it is difficult to detect the pupil, it may be determined that the object person turns sideways or is asleep. Accordingly, in a case where it is difficult to detect the pupil, it is possible to adjust the posture of the object person by, for example, issuing an alert.

In addition, according to the present invention, it is not necessary to construct three-dimensional data from an entire face image in such a manner as described in PTL 1. Accordingly, compared with the related art, it is possible to reduce a control load (calculation load) on a device, and it is possible to smoothly perform up to measurement of the biological information.

In the present invention, it is desirable that the image capturing unit includes an imaging element and a light-emitting element capable of irradiating the object person with light. From this, it is possible to adequately detect the pupil of the object person.

In addition, in the present invention, it is desirable that the imaging elements are provided. From this, based on trigonometry, it is possible to obtain a distance between the image capturing unit and the object person.

In addition, in the present invention, it is desirable that the light-emitting element includes a first light-emitting element that radiates an infrared ray having a first wavelength and a second light-emitting element that radiates an infrared ray having a second wavelength longer than the first wavelength, a bright pupil image is captured under a condition that the infrared ray having the first wavelength is radiated, a dark pupil image is captured while the infrared ray having the second wavelength is radiated or the infrared ray is not radiated, and the pupil area is detected based on a difference image between the bright pupil image and the dark pupil image, in the detection unit. From the above, it is possible to easily and stably detect the pupil area. In addition, for example, the dark pupil image may be used as an image for identifying the skin area. In addition, utilization of the infrared ray enables the biological information to be detected during the night.

In addition, in the present invention, it is desirable that it is possible to detect a corneal reflection image, in the detection unit. In a face image obtained in a state of being irradiated with light, the corneal reflection image is reflected. Therefore, it is possible to adequately and easily obtain the corneal reflection image. In addition, using the corneal reflection image, it becomes possible to sense the visual line or the like of the object person.

In addition, it is desirable that, in the luminance acquisition unit, an average value of luminances of the skin area image-captured using the imaging elements or different wavelengths is acquired and input to the biological measurement unit. By acquiring and making the average value of the luminances of the skin area available for measuring the biological information in this way, it is possible to execute measurement of the biological information with a high degree of accuracy.

In addition, it is desirable that pupil tracking is executed in the detection unit. By performing the pupil tracking, it is possible to perform measurement of the visual line or the like of the object person with a high degree of accuracy.

In addition, in the detection unit, it is possible to detect the visual line of the object person.

In addition, in the detection unit, it is possible to detect a face direction of the object person.

In addition, it is desirable that a luminance of the skin area located below the pupil area is acquired in the luminance acquisition unit. Unlike an area located above the pupil area, in the skin area located below the pupil area, a change in the skin area, due to a blink, is less likely to occur, and it is possible to stably obtain the luminance.

In addition, in the present invention, it is desirable that in a case where the pupil areas of two eyes of the object person are acquired, the luminance acquisition unit acquires the luminance of the skin area. In a case where it is possible to obtain the pupil area of only one eye, it may be determined that the object person does not face forward (an image capturing unit side). In such a case, it is possible to invite attention to looking away. A case where the pupil areas of two eyes are obtained corresponds to a state in which the object person faces forward. In such a case, by acquiring the luminance of the skin area and measuring the biological information, it is possible to adjust the posture of the object person and stably measure the biological information.

In addition, in the present invention, it is possible to adopt a configuration in which the biological information measurement device is arranged within a vehicle. By arranging the biological information measurement device within a vehicle, it is possible to obtain the biological information of a driver during driving, and, based on that, it is possible to perform drive assist or the like.

In addition, an input device in the present invention includes the biological information measurement device described above and an input operation unit, wherein a predetermined input operation or predetermined information transmission is executed based on information from the biological information measurement device.

In the present invention, it is desirable that the input operation is executed by predicting a behavior of the object person.

In addition, it is desirable that the input operation is made available for drive assist.

In the present invention, based on pieces of information (the biological information, pupil information, visual line information, and so forth) from the biological information measurement device, the behavior of the object person is predicted, and based on this prediction, it is possible to perform a predetermined input operation. From this, even if an operation body such as a hand does not directly touch an operation unit, it is possible to speedily execute an input operation. In a case of a configuration in which the biological information measurement device of the present invention is arranged in, for example, a vehicle, it is possible to determine ambient brightness from a pupil size and accelerate the response of automatic lighting. In addition, it is possible to determine whether or not falling asleep, and in a case where it is determined as falling asleep, it is possible to draw attention with sound, for example, and it is possible to enhance safety.

### Advantageous Effects of Invention

According to the present invention, since the skin area made available for sensing a luminance is identified based on the pupil area, it is possible to stably obtain the skin area and it is possible to stably obtain the luminance of the skin area with a high degree of accuracy. Therefore, it is possible to stably obtain, with a high degree of accuracy, the biological information obtained based on the luminance of the skin area. It is possible to correctly detect the pupil without fail unlike the other part of the face of the object person. Therefore, in contrast, in a case where it is difficult to detect the pupil, it may be determined that the object person turns sideways or is asleep. Accordingly, in a case where it is difficult to detect the pupil, it is possible to adjust the posture of the object person by, for example, issuing an alert.

In addition, according to the present invention, it is not necessary to construct three-dimensional data from an entire face image in such a manner as described in PTL 1. Accordingly, compared with the related art, it is possible to reduce a control load (calculation load) on a device, and it is possible to smoothly perform up to measurement of the biological information.

### Brief Description of Drawings

[Fig. 1] Fig. 1 is a pattern diagram illustrating a state in which a driver (object person) is image-captured by a near-infrared camera (image capturing unit).
[Fig. 2] Fig. 2(a) is a front view of the near-infrared camera (image capturing unit) in the present embodiment and Fig. 2(b) is a side view illustrating an internal structure of the near-infrared camera.
[Fig. 3] Fig. 3(a) is a bright pupil image, Fig. 3(b) is a dark pupil image, and Fig. 3(c) is a pattern diagram illustrating a difference image between the bright pupil image and the dark pupil image.
[Fig. 4] Fig. 4(a) is a pattern diagram in which a surrounding area of a pupil area is identified as a skin area for measuring a luminance and Fig. 4(b) is a pattern diagram in which only an area located below the pupil area is identified as a skin area for measuring a luminance, within the skin area illustrated in Fig. 4(a).
[Fig. 5] Fig. 5 is a block diagram of a biological information measurement device in the present embodiment and an input device utilizing this.
[Fig. 6] Fig. 6(a) is a flowchart diagram from acquisition of the bright pupil image and the dark pupil image to activation of a predetermined input operation, Fig. 6(b) is a flowchart diagram from pupil tracking to calculation of a visual line vector, and Fig. 6(c) is a flowchart diagram from nostril detection to face direction detection.
[Fig. 7] Fig. 7 is a relationship diagram illustrating a relationship between a pupil image, a corneal reflection image, and a visual line calculation.
[Fig. 8] Fig. 8 is a pattern diagram illustrating a corneal reflection image.
[Fig. 9] Figs. 9(a) to 9(d-3) are explanatory diagrams for explaining an algorithm for visual line calculation and Fig. 9(e) is a graph illustrating a relationship between a distance r between a pupil center and corneal reflection and an angle θ between a camera pupil vector and a visual line vector.
[Fig. 10] Fig. 10 is a pattern diagram for explaining a method for measuring a face direction.
[Fig. 11] Fig. 11 is a pattern diagram illustrating a relationship between two near-infrared cameras and a viewpoint.
[Fig. 12] Fig. 12 is a flowchart diagram of an input device utilizing a biological information measurement device in another embodiment (a second invention).
[Fig. 13] Fig. 13 is a pattern diagram in which a surrounding area of a corneal reflection image is identified as a skin area for measuring a luminance.

### Description of Embodiments

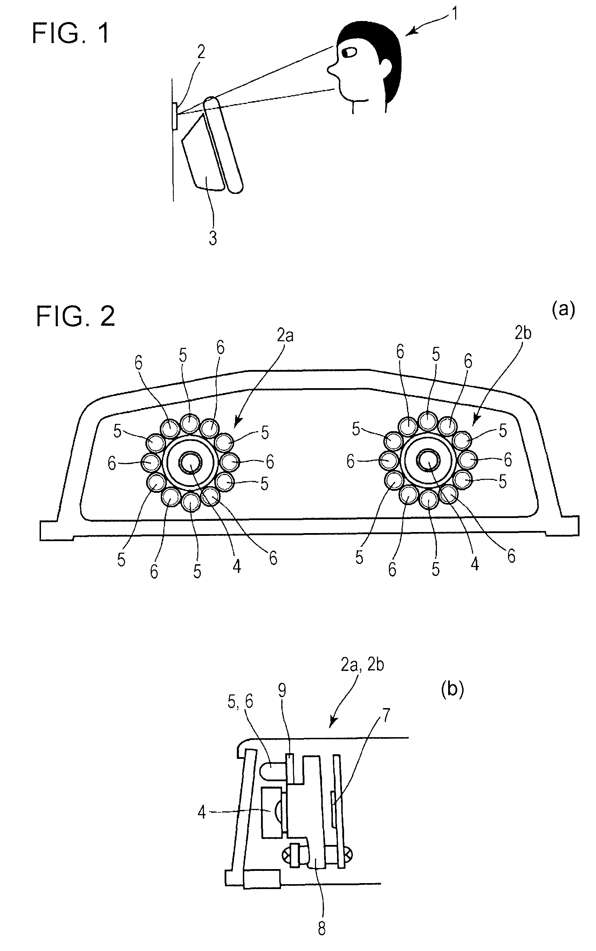

Fig. 1 is a pattern diagram illustrating a state in which a driver (object person) is image-captured by a near-infrared camera (image capturing unit). In the present embodiment, as illustrated in Fig. 1, the image of a driver 1 who sits in a driver seat in a vehicle is captured using a near-infrared camera 2 and the pupils of the driver 1 are detected using the image data thereof.

The near-infrared camera 2 is arranged in front of the driver seat and arranged on, for example, an instrument panel. Alternatively, the near-infrared camera 2 may be installed in a portion of a steering supporting section 3.

As illustrated in Fig. 2(a), two near-infrared cameras 2a and 2b are provided. As illustrated in Figs. 2(a) and 2(b), the near-infrared cameras 2a and 2b each include a lens 4, a plurality of first light-emitting elements 5, a plurality of second light-emitting elements 6, an imaging element (sensor substrate) 7 located posterior to the lens 4, and a chassis 8 that supports the lens 4, the individual light-emitting elements 5 and 6, and the imaging element 7.

Here, the first light-emitting elements 5 are 870-nm LEDs and the second light-emitting elements 6 are 940-nm LEDs. Note that the wavelengths are just examples and wavelengths other than those may be adopted.

The individual light-emitting elements 5 and 6 are mounted in a board 9, and an LED board in which the light-emitting elements 5 and 6 are arranged on the board 9 and the imaging element 7 are arranged in parallel. In addition, the two near-infrared cameras 2a and 2b are used while being synchronized with each other.

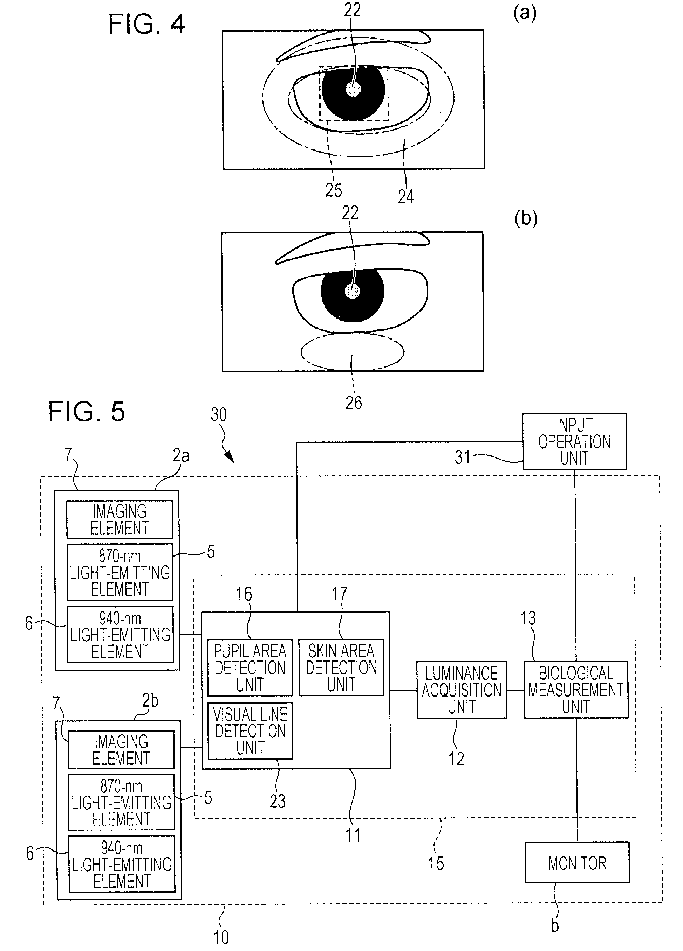

As illustrated in a block diagram of Fig. 5, a biological information measurement device 10 in the present embodiment includes the near-infrared cameras 2a and 2b, a detection unit 11, a luminance acquisition unit 12, a biological measurement unit 13, and a monitor 14. In addition, a control unit 15 is configured by putting together the detection unit 11, the luminance acquisition unit 12, and the biological measurement unit 13.

As illustrated in Fig. 5, in the detection unit 11, a pupil detection unit 16, a skin area detection unit 17, and a visual line detection unit 23 are provided.

In the pupil detection unit 16, a difference image between a bright pupil image and a dark pupil image is created. The bright pupil image and the dark pupil image will be described below.

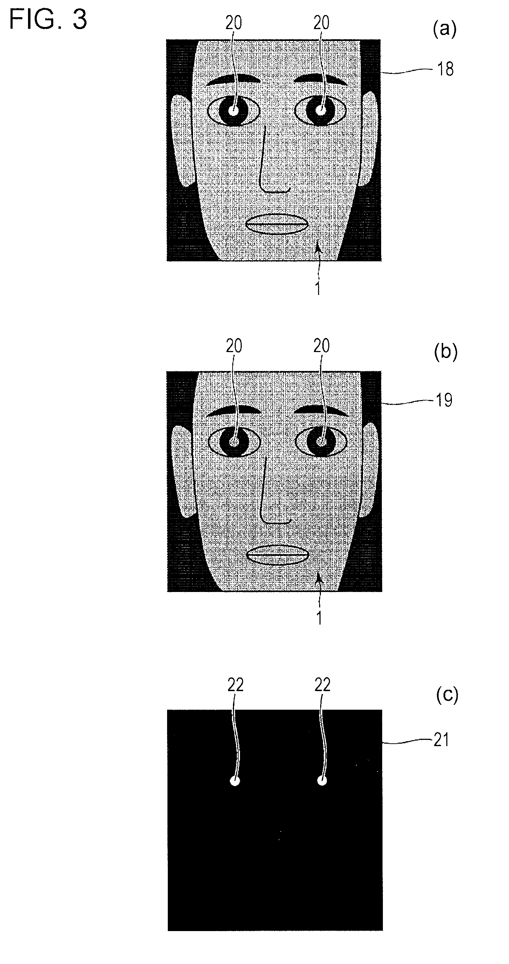

Fig. 3(a) is a bright pupil image 18 of the driver (object person) 1, and Fig. 3(b) is a dark pupil image 19 of the driver (object person) 1.

First, in a state in which, using the first light-emitting elements 5, the face of the driver 1 is irradiated with near-infrared rays whose wavelengths are 870 nm, a face image is captured by the imaging element 7. In the face image captured in this way, the bright pupil image 18 in which pupils 20 are image-captured so as to be significantly brighter than the other part is obtained as illustrated in Fig. 3(a). On the other hand, in a state in which, using the second light-emitting elements 6, the face of the driver 1 is irradiated with near-infrared rays whose wavelengths are 940 nm, a face image is captured using the imaging element 7. In the face image captured in this way, the dark pupil image 19 in which the pupils 20 are image-captured so as to be darker than the bright pupil image 18 in Fig. 3(a) is obtained as illustrated in Fig. 3(b). The wavelengths of the light-emitting elements are set to respective wavelengths for obtaining bright pupils and dark pupils.

The capturing of the bright pupil image 18 and the capturing of the dark pupil image 19 are performed in a time division manner. In addition, since, in the present embodiment, there are the two near-infrared cameras 2a and 2b, the bright pupil image 18 and the dark pupil image 19 are acquired in each of the near-infrared camera 2a and the near-infrared camera 2b.

In the pupil detection unit 16 illustrated in Fig. 5, differences image between the bright pupil images 18 and the dark pupil images 19 are created. One of difference images 21 is illustrated in Fig. 3(c). It is possible for each of the difference images 21 to be obtained by, for example, subtracting the luminance values of the pixels of the corresponding dark pupil image 19 from the luminance values of the respective pixels of the corresponding bright pupil image 18.

As illustrated in Fig. 3(c), one of the difference images 21 is left as an image in which the pupils 20 of the driver (object person) 1 are brighter than the peripheries thereof. Bright portions illustrated in Fig. 3(c) are identified as pupil areas 22. Here, as illustrated in Fig. 3(c), it is possible to acquire the two pupil areas 22. The bright pupil images and the dark pupil images described above correspond to those explained using a method similar to Japanese Unexamined Patent Application Publication No. 2008-125619. A case where it is assumed that, as a result of obtaining a pupil area using the above-mentioned method, it is possible to obtain only one pupil area 22 corresponds to a state in which it is possible to acquire obtain the pupil area of one eye, and it may be determined that the driver 1 does not face forward (an image capturing unit side). In such a case, it is possible to invite attention to looking away. Only after it becomes possible to acquire the pupil areas 22 of two eyes, it is possible to control so as to measure biological information.

While, in the above description, face images captured by radiating near-infrared rays whose wavelengths are 940 nm are set as the dark pupil images 19 used for obtaining the difference images 21, face images captured by, for example, not radiating light such as infrared rays may be set as the dark pupil images 19.

Next, in the skin area detection unit 17, skin areas made available for measuring the biological information are acquired, based on the pupil areas 22.

Fig. 4(a) is a pattern diagram that magnifies the vicinity of an eye illustrated in, for example, one of the dark pupil images 19. Since the pupil areas 22 are identified based on Fig. 3(c), a skin area 24 for acquiring a luminance is identified around each of the pupil areas 22. The skin areas 24 are identified at positions located predetermined distances away from the pupil areas 22 and located outside thereof.

In addition, an area 25 of an eye (a black eye portion) may be identified from each of the pupil areas 22, and the skin area 24 for acquiring a luminance may be identified from the corresponding area 25 of an eye.

If it is possible to identify the pupil areas 22, it is possible to estimate the area of a corresponding eye, based on the luminance or the like of an image of the periphery of each of the pupil areas 22, and accordingly, it is possible to keep a load on the detection unit 11 at a low level at the time of detecting the skin areas 24 located away from the pupil areas 22.

Note that binarization processing, labeling processing, screening processing, and so forth are arbitrarily executed in the detection unit 11.

In the luminance acquisition unit 12 illustrated in Fig. 5, the luminances of the skin areas 24 are acquired. At this time, the average values of luminance obtained from, for example, respective images captured in the near-infrared camera 2a and the near-infrared camera 2b or images captured at different wavelengths are continuously acquired. In addition, at this time, it is not necessary to acquire the luminances of all the pixels of each of the skin areas 24. In a case where a luminance is acquired from one portion of each of the skin areas 24 in this way, it is desirable that, as illustrated in Fig. 4(b), the luminances of the skin areas 26 on located below the respective pupil areas 22 are acquired. An area located above each of the pupil areas 22 is an eyelid side and a blink makes it difficult to obtain a stable luminance. Therefore, by acquiring the luminances of the skin areas 26 on eye bag sides located below the respective pupil areas 22, it is possible to stably acquire luminances.

In the biological measurement unit 13 illustrated in Fig. 5, the biological information of the driver (object person) 1 is measured from the luminance of each of skin areas obtained by the luminance acquisition unit 12. The measurement of the biological information prepares the value of change in luminance of each of the skin areas during a given period of time. Next, a low-frequency noise due to a body movement or the like is removed, and an independent signal is extracted using an independent component analysis method. There is a method in which the power spectrum of this independent signal is calculated and a signal having a frequency peak in the vicinity of a heart rate or a breathing rate is acquired, thereby setting this as an output signal. However, here the biological information or a measuring method therefor is not specifically limited.

As illustrated in Fig. 5, the input device 30 includes the biological information measurement device 10 and an input operation unit 31. Here, the input operation unit 31 may double as the monitor 14. Therefore, as an configuration element of the biological information measurement device 10, the monitor 14 may exist or may be omitted.

The biological information is transmitted from the biological measurement unit 13 to the input operation unit 31. Alternatively, pieces of information (pupil information, visual line information, and so forth) from the detection unit 11 illustrated in Fig. 5 are transmitted thereto. In the input operation unit 31, based on these pieces of information transmitted from the biological information measurement device 10, a predetermined input operation or predetermined information transmission is executed. What input operation and what information transmission are performed will be described later.

In the input operation unit 31, it is possible to predict the behavior of the driver (object person) 1 and execute an input operation. By focusing attention on, for example, the visual line of the driver 1, behavior prediction is performed based on the direction of the visual line, and an input operation based on the behavior prediction is executed.

It is preferred that the input device 30 illustrated in Fig. 5 in the present embodiment is mounted within a vehicle and an input operation based on the pieces of information from the biological information measurement device 10 illustrated in Fig. 5 is made available for drive assist. For example, to accelerate the response of automatic lighting by judging from the sizes of the pupils of the driver 1 may be cited.

In the present embodiment, the skin areas 24 and 26 are identified based on the pupil areas 22. Therefore, it is possible to stably obtain the skin areas 24 and 26, and it is possible to stably obtain the luminances of the skin areas 24 and 26 with a high degree of accuracy. Accordingly, it is possible to stably obtain, with a high degree of accuracy, the biological information obtained based on the luminances. It is possible to correctly detect the pupils without fail unlike the other part of the face of the object person. Therefore, in contrast, in a case where it is difficult to detect a pupil, it may be determined that the object person turns sideways or is asleep. Accordingly, in a case where it is difficult to detect a pupil, it is possible to draw the object person's attention to facing forward (a near-infrared camera 2 side) with eyes open.

In addition, according to the present embodiment, it is not necessary to construct three-dimensional data from an entire face image in such a manner as described in PTL 1. Accordingly, compared with the related art, it is possible to reduce a control load (calculation load) on a device, and it is possible to smoothly perform up to measurement of the biological information.

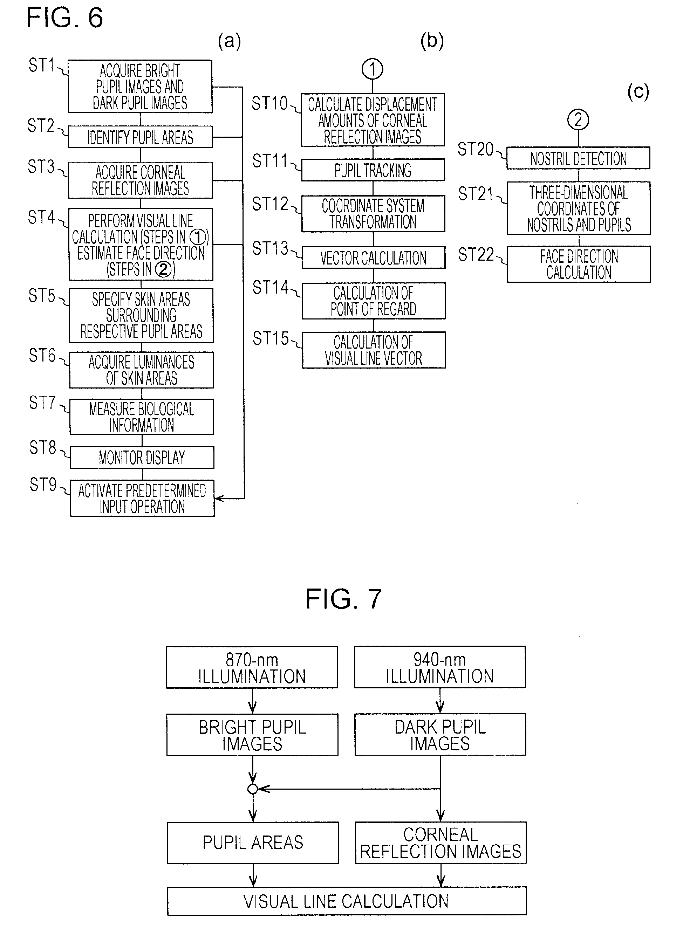

Next, using a flowchart illustrated in Fig. 6, steps from acquisition of an image to activation of an input operation will be described.

In a step ST1 in Fig. 6(a), the bright pupil images 18 and the dark pupil images 19 are acquired (see Figs. 3(a) and 3(b)). As illustrated in Fig. 7, by image-capturing under a condition that near-infrared rays whose wavelengths are 870 nm are radiated by the first light-emitting elements 5, it is possible to obtain the bright pupil images 18. In addition, by image-capturing under a condition that near-infrared rays whose wavelengths are 940 nm are radiated by the second light-emitting elements 6, it is possible to obtain the dark pupil images 19. Note that the dark pupil images 19 may be captured by radiating no light from the light-emitting elements.

Next, in a step ST2 in Fig. 6(a), the pupil areas 22 are identified based on the respective difference images 21 between the bright pupil images 18 in Fig. 3(a) and the dark pupil images 19 in Fig. 3(b).

Next, as illustrated in Fig. 6(a), in a step ST3, corneal reflection images are acquired. As illustrated in Fig. 7, it is possible to acquire the corneal reflection images, based on the dark pupil images 19 captured under the condition that near-infrared rays whose wavelengths are, for example, 940 nm are radiated.

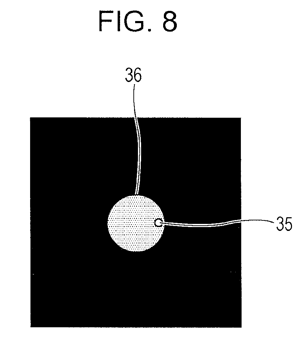

In Fig. 8, one of corneal reflection images 35 is illustrated. Fig. 8 magnifies and illustrates the pupil portion of one of dark pupil images. As illustrated in the dark pupil image, it turns out that one of the corneal reflection images 35 brighter than a corresponding pupil 36 reflected darkly is reflected. The corneal reflection images 35 are the reflection images of a light source reflected from corneas of the driver 1.

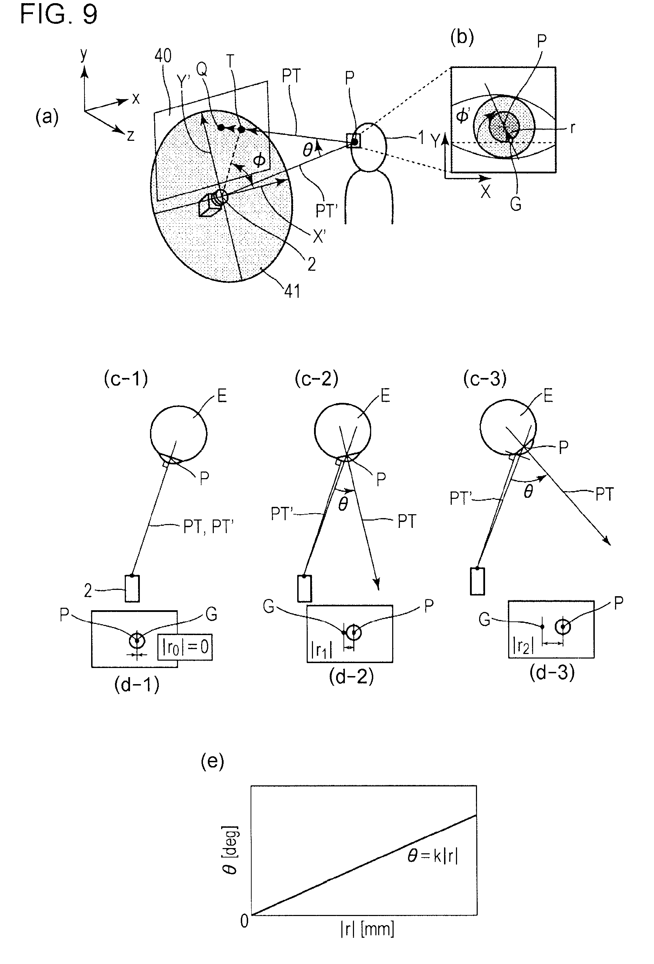

Subsequently, in a step ST4 illustrated in Fig. 6(a), visual line calculation is performed. As illustrated in Fig. 7, it is possible to perform the visual line calculation using the pupil areas and the corneal reflection images. An algorithm for the visual line calculation will be described using Fig. 9. Here, the algorithm for the visual line calculation illustrated in Fig. 9 will be described using a method similar to WO2012-20760.

As illustrated in Fig. 9(a), it is assumed that a visual target plane 40 exists in front of the driver (object person) 1. This visual target plane 40 is, for example, a display. It is assumed that this visual target plane 40 is a surface parallel to an X-Y plane. A symbol P is the pupil center of the driver (object person) 1 and PT is a visual line vector. Q is a point of regard.

The corresponding near-infrared camera 2 is mounted in parallel to a virtual viewpoint plane 41 serving as a surface parallel to an X'-Y' plane. In other words, a direction perpendicular to the virtual viewpoint plane 41 is the optical axis direction of a camera. T is a point of regard on the virtual viewpoint plane 41. PT' is a camera pupil vector.

As illustrated in Fig. 9(a), an angle between the visual line vector PT and the camera pupil vector PT' is θ. In addition, an angle between a direction from a camera center to the point of regard T on the virtual viewpoint plane 41 and an X'-axis is φ.

Fig. 9(b) is a pattern diagram of one of pupil periphery images. G is one of the corneal reflection images. An angle between a line linearly connecting one of the corneal reflection images G with one of the pupil centers P and an X-axis is φ'.

E illustrated in each of Figs. 9(c-1) to 9(c-3) illustrates one of eyeballs of the driver (object person) 1 in such a manner as a pattern diagram. In Figs. 9(c-1) to 9(c-3), the directions of the eyeball are different. In Fig. 9(c-1), the directions of the visual line vector PT and the camera pupil vector PT' coincide with each other. In other words, θ illustrated in Fig. 9(a) is zero. At this time, in the image of Fig. 9(d-1), the pupil center P and the corneal reflection image G coincide with each other. In other words, an interval |r₀| = 0 between the corresponding pupil center P and the corresponding corneal reflection image G is satisfied.

Next, in Fig. 9(c-2), the visual line vector PT and the camera pupil vector PT' do not coincide with each other, and an angle θ is generated between the visual line vector PT and the camera pupil vector PT'. At this time, in the image of Fig. 9(d-2), an interval |r₁| is generated between the corresponding pupil center P and the corresponding corneal reflection image G.

In addition, in Fig. 9(c-3), the angle θ between the visual line vector PT and the camera pupil vector PT' is larger than that in Fig. 9(c-2). At this time, in the image of Fig. 9(d-3), an interval |r₂| is generated between the corresponding pupil center P and the corresponding corneal reflection image G, and this interval |r₂| is larger than the interval |r₁| illustrated in Fig. 9(d-2).

Here, a relationship between (θ,φ) illustrated in Figs. 9(a) and 9(c) and (|r|,φ') illustrated in Figs. 9(b) and 9(d) is a one-to-one correspondence.

In other words, the interval (distance) |r| between the corresponding pupil center P and the corresponding corneal reflection image G increases with an increase in the distance of the visual line from a camera optical axis (with an increase in θ). Accordingly, between θ and |r|, there is a relationship illustrated in Fig. 9(e). Based on this relationship, it is possible to estimate the visual line.

Specifically, in a step ST10 illustrated in Fig. 6(b) (the specific step of the step ST4 in Fig. 1(a)), the displacement amounts of the corneal reflection images are calculated. The displacement amount of each of the corneal reflection images is indicated as the interval (distance) |r| between the corresponding pupil center P and the corresponding corneal reflection image G, illustrated in each of Figs. 9(d-1) to 9(d-3).

Note that, by executing pupil tracking based on improvement of resolution, it is possible to determine the corresponding pupil center P (step ST11).

In a step ST12 in Fig. 6(b), the corresponding pupil center P and the corresponding corneal reflection image G are transformed to an X-Y coordinate system. Subsequently, in a step ST13 illustrated in Fig. 6(b), a vector of pupil center P-corneal reflection image G is calculated, and based on trigonometry as illustrated in the relationship diagrams in Figs. 9(a) and 9(b), the point of regard T on the virtual viewpoint plane 41 is calculated (step ST14). In addition, in a step ST15, the visual line vector PT is calculated.

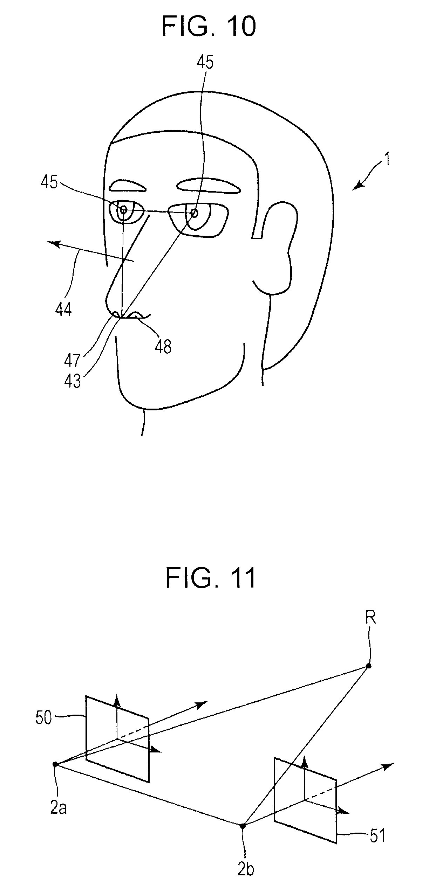

In addition, in the step ST4 in Fig. 6(a), a face direction may be estimated. Fig. 10 is a perspective view when the face of the driver (object person) 1 is viewed from an oblique front side. From the positions of pupils 45 detected by the steps ST1 and ST2 in Fig. 6(a), the existence range of the nostrils is estimated, and from the existence range (area), right and left nostrils 47 and 48 are detected (step ST20 in Fig. 6(c)). At this time, only one nostril may be detected. Note that if the nostrils are detected in a previous image (previous frame), nostril positions are estimated from the nostrils positions of the previous image and tracked, for a subsequent image. In addition, in a case where no nostrils are detected in the previous image, a search for nostrils is executed from an entire image.

By the way, as for the detection of the nostrils 47 and 48, it is possible to roughly determine the existence range of the nostrils from the positions of the pupils 45, and it is possible to confirm the nostrils, based on luminance measurement within the range. In addition, by performing the binarization processing on an image, it becomes easy to detect the nostrils.

Subsequently, based on stereo calculation, the three-dimensional coordinates of a midpoint 43 connecting the nostrils 47 and 48 and the midpoint pupils 45 are calculated (step ST21 illustrated in Fig. 6(c)).

In addition, as illustrated in Fig. 10, a line 44 normal to a triangle obtained by connecting the pupils 45 and 40 located on both right and left sides and the midpoint 43 with one another is obtained, and this normal line 44 is estimated as the direction of the face (step ST22 in Fig. 6(c)).

Subsequently, in a step ST5 in Fig. 6(a), the skin areas 24 surrounding the respective pupil areas 22 are identified (see Fig. 4). As illustrated in Fig. 4(b), the respective skin areas 26 may be portions of the peripheries of the respective pupil areas 22, and in that case, it is preferred that the luminances of the skin areas 26 located below the respective pupil areas 22 are selected. In addition, in a step ST6 in Fig. 6(a), the luminances of the skin areas 24 and 26 are acquired. It is preferred that the luminances are average values. The average value of luminances may be obtained using the average of the luminances of a corresponding skin area image-captured by a plurality of imaging elements or the average of the luminances of a corresponding skin area image-captured by different wavelengths. In addition, in a step ST7, based on the luminances of skin areas, biological information is measured. According to the present embodiment, it is possible to obtain pieces of biological information such as a heart rate, a breathing rate, and a pulse rate.

In addition, in a step ST8 illustrated in Fig. 6(a), the biological information is sent to the monitor 14 and displayed on the monitor 14. The monitor 14 is provided in, for example, an intermediate portion between the near-infrared cameras 2a and 2b illustrated in Fig. 2(a). Alternatively, the monitor 14 may be an operation panel or the like that configures the input device 30. A touch panel or the like of a car navigation device arranged in a center console corresponds to the operation panel.

In addition, as illustrated in a step ST9 in Fig. 6(a), the biological information is transmitted to the input operation unit 31 that configures the input device 30. In addition, pieces of information such as the face images (the bright pupil images and the dark pupil images) acquired in the step ST1, the pupil areas acquired in the step ST2, the corneal reflection images acquired in the step ST3, and the visual line direction and the face direction in Fig. 6(c), acquired in the step ST4, are transmitted to the input operation unit 31. The number of pieces of information to be transmitted to the input operation unit 31 may be one or two or more.

In the input operation unit 31, based on the information from the biological information measurement device 10, a predetermined input operation or predetermined information transmission is executed. Hereinafter, a specific example will be illustrated.

First, based on the visual line direction obtained in the step ST4 in Fig. 6(a), it is possible to perform input assistance (drive assist). For example, as an input based on the visual line detection, sound volume or the like is allowed to be set using the visual line while a steering switch is pressed. In addition, as selection based on the visual line, selection of a control device or the like is allowed to be performed using the visual line. In addition, as direction indicator assistance, a blinker is turned on and a backward left screen is displayed on the monitor, at the time of viewing, for example, a sideview mirror. In addition, if a blinker is turned on without viewing a sideview mirror, warning sound is emitted. In addition, if, after warning display is performed on the monitor, the driver 1 views that, alert cancellation is executed. In addition, if the frequency of looking around decreases, an alert such as "please pay attention to surroundings" or "please take a break" may be issued or an action such as vibrating a sheet may be taken.

In addition, as an input based on the pupil detection, ambient brightness is determined from the pupil sizes and the response of automatic lighting is accelerated. In addition, based on the pupil sizes, a mirror angle is controlled. In addition, based on the pupil sizes, the transmittance of a windshield is adjusted.

In addition, in a case where it is difficult to confirm the pupils, there is a possibility of falling asleep. Therefore, after displaying an alert on a meter or the like, it is possible to confirm, based on the subsequent pupil detection, whether that display is viewed.

In addition, lips of mouth are detected from the positions of the pupils, and based on the movements of the lips of mouth, it is possible to improve the degree of accuracy of a sound input.

If looking away or looking aside is detected in, for example, a case where it is possible to confirm the pupil of only one eye, warning sound is emitted or visual line navigation is executed.

In addition to these, based on the visual line direction, it is possible to adjust the display height of a meter or the like and it is possible to cause a seat height or a handle height to be automatically adjusted. In addition, based on a distance between right and left pupils, distances from nostrils, or the shapes of nostrils, it is possible to execute personal authentication.

In the present embodiment, the two near-infrared cameras 2a and 2b are provided. While, in the present embodiment, the number of the near-infrared cameras is set to one, the near-infrared cameras 2a and 2b whose number is two or more (at least two) are installed, thereby enabling a distance from the driver (object person) 1 to be obtained using the trigonometry. As illustrated in Fig. 11, a visibility based on an image 50 that reflects a target object R using the near-infrared camera 2a and a visibility based on an image 51 that reflects a target object R using the near-infrared camera 2b are different from each other. From this difference between visibilities, it is possible to identify the position of the target object R using the trigonometry. Accordingly, in the present embodiment, using the trigonometry, it is possible to obtain a distance from the driver (object person) 1, from images captured by the near-infrared cameras 2a and 2b.

In addition, in the embodiment of Fig. 6(a), the steps ST3, ST4, ST8, and ST9 are not essential steps but selective steps.

In the above-mentioned embodiment (the first embodiment), the pupil areas are identified in the step ST2 in Fig. 6(a), and the skin areas for measuring luminances are identified based on the pupil areas.

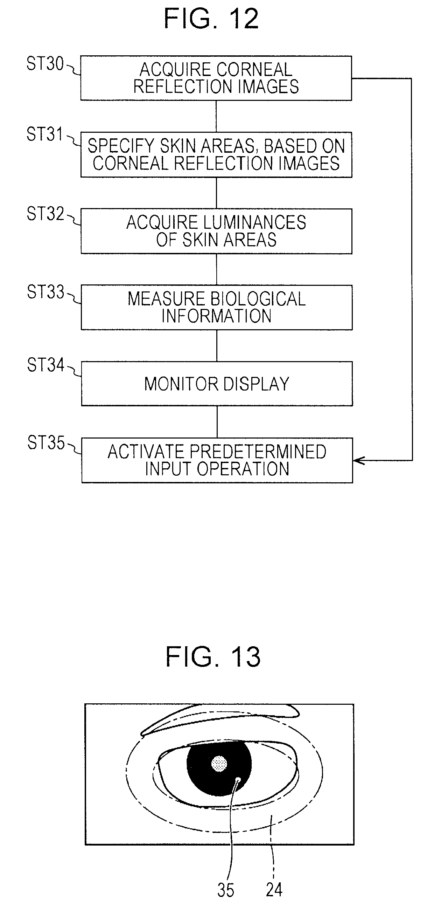

In contrast, in a second embodiment illustrated in Fig. 12, in a step ST30, the corneal reflection images 35 are acquired, and in a step ST31, the skin areas 24 are identified based on the corneal reflection images 35. As illustrated in Fig. 7, the corneal reflection images 35 may be obtained based on, for example, the dark pupil images 19.

Unlike the first embodiment, in this second embodiment, it is not essential to acquire the pupil areas 22. However, in place thereof, it is essential to acquire the corneal reflection images 35. In addition, as illustrated in Fig. 13, the skin areas 24 are identified around the respective corneal reflection images 35. The skin areas 24 are set at positions located a predetermined distance away from the respective corneal reflection images 35. Based on the luminances or the like of images, it is possible to identify the areas of eyes of the object person from the corneal reflection images 35, and it is possible to set the skin areas 24 around the identified eyes.

A step ST32 illustrated in Fig. 12 corresponds to the step ST6 in Fig. 6(a), a step ST33 in Fig. 12 corresponds to the step ST7 in Fig. 6(a), a step ST34 in Fig. 12 corresponds to the step ST8 in Fig. 6(a), and a step ST35 in Fig. 12 corresponds to the step ST9 in Fig. 6(a).

Note that, in the second embodiment illustrated in Fig. 12, as illustrated in the steps ST1 and ST2 in Fig. 6(a), it is possible to identify the pupil areas 22, based on the respective difference images between the bright pupil images 18 and the pupil images 19. In such a case, it becomes possible to perform the visual line calculation and so forth, as illustrated in Fig. 7.

While being not limited to vehicle applications, the biological information measurement device 10 of the present embodiment and the input device 30 utilizing that are used for vehicle applications, thereby enabling the biological information of the driver to be obtained during driving, and, based on that, it is possible to perform drive assist or the like.

By tracking, for example, pupils, the behavior prediction may be determined based on the tracking result thereof.

In addition, based on pieces of information (the pupil information, the visual line information, the biological information, and so forth) obtained from the biological information measurement device 10, it is possible to determine whether or not, for example, falling asleep. In addition, in such a case, it is possible to invite an early attention using sound or the like, and by predicting the behavior of the driver (object person), it is possible to execute a predetermined input operation.

While, in the above-mentioned embodiment, the driver is set as an object person whose biological information is to be measured, an occupant in a passenger seat or the like may be set as an object person without being limited to the driver.

### Reference Signs List

- G, 35: corneal reflection image
- P: pupil center
- PT: visual line vector
- 1: driver
- 2, 2a, 2b: near-infrared camera
- 5: first light-emitting element
- 6: second light-emitting element
- 7: imaging element
- 10: biological information measurement device
- 11: detection unit
- 12: luminance acquisition unit
- 13: biological measurement unit
- 14: monitor
- 16: pupil detection unit
- 17: skin area detection unit
- 18: bright pupil image
- 19: dark pupil image
- 20, 45: pupil
- 21: difference image
- 22: pupil area
- 24, 26: skin area
- 30: input device
- 31: input operation unit
- 47, 48: nostril

## Claims

1. A biological information measurement device comprising:
an image capturing unit capable of detecting a pupil of an object person;
a detection unit capable of detecting a pupil area from image data obtained by the image capturing unit;
a luminance acquisition unit that acquires a luminance of a skin area serving as at least a portion of the periphery of the pupil area; and
a biological measurement unit that measures biological information of the object person from the luminance of the skin area.

2. The biological information measurement device according to Claim 1, wherein
the image capturing unit includes an imaging element and a light-emitting element capable of irradiating the object person with light.

3. The biological information measurement device according to Claim 2, wherein
the imaging elements are provided.

4. The biological information measurement device according to Claim 2, wherein
the light-emitting element includes a first light-emitting element that radiates an infrared ray having a first wavelength and a second light-emitting element that radiates an infrared ray having a second wavelength longer than the first wavelength,
a bright pupil image is captured under a condition that the infrared ray having the first wavelength is radiated, and a dark pupil image is captured while the infrared ray having the second wavelength is radiated or the infrared ray is not radiated, and
the pupil area is detected based on a difference image between the bright pupil image and the dark pupil image, in the detection unit.

5. The biological information measurement device according to Claim 2, wherein
it is possible to detect a corneal reflection image, in the detection unit.

6. The biological information measurement device according to Claim 1, wherein
in the luminance acquisition unit, an average value of luminances of the skin area image-captured using the imaging elements or different wavelengths is acquired and input to the biological measurement unit.

7. The biological information measurement device according to Claim 1, wherein
pupil tracking is executed in the detection unit.

8. The biological information measurement device according to Claim 1, wherein
a visual line of the object person is detected in the detection unit.

9. The biological information measurement device according to Claim 1, wherein
a face direction of the object person is detected in the detection unit.

10. The biological information measurement device according to Claim 1, wherein
a luminance of the skin area located below the pupil area is acquired in the luminance acquisition unit.

11. The biological information measurement device according to Claim 1, wherein
in a case where the pupil areas of two eyes of the object person are acquired, the luminance acquisition unit acquires the luminance of the skin area.

12. The biological information measurement device according to Claim 1, wherein
the biological information measurement device is arranged within a vehicle.

13. An input device comprising: the biological information measurement device according to Claim 1; and an input operation unit, wherein
a predetermined input operation or predetermined information transmission is executed based on information from the biological information measurement device.

14. The input device according to Claim 13, wherein
the input operation is executed by predicting a behavior of the object person.

15. The input device according to Claim 13, wherein
the input operation is made available for drive assist.
